# EUROPEAN PATENT SPECIFICATION

(11) **EP 3 733 222 B1**
(45) Date of publication and mention of the grant of the patent: **23.08.2023**
(21) Application number: 18897186.5
(22) Date of filing: 31.10.2018
(51) Int. Cl.: A61L 31/02, A61L 31/06, A61L 31/10, A61L 31/14, A61L 31/16, A61L 27/04, A61L 27/34, A61L 27/50, A61L 27/54, A61L 27/58

(54) **ABSORBABLE IRON-BASED IMPLANTABLE DEVICE**
ABSORBIERBARE IMPLANTIERBARE VORRICHTUNG AUF EISENBASIS
DISPOSITIF IMPLANTABLE À BASE DE FER RÉSORBABLE

(30) Priority: 25.12.2017 CN 201711424079
(43) Date of publication of application: 04.11.2020
(73) Proprietor: Biotyx Medical (Shenzhen) Co., Ltd., Shenzhen, Guangdong 518000 (CN)
(72) Inventor: LIN, Wenjiao, Shenzhen, Guangdong 518057 (CN); CHEN, Liping, Shenzhen, Guangdong 518057 (CN); QIN, Li, Shenzhen, Guangdong 518057 (CN)
(74) Representative: Prinz & Partner mbB
(86) International application number: PCT/CN2018/112861
(87) International publication number: WO 2019/128453

(56) References cited:
- WO-A2-2008/034031
- CN-A- 102 307 469
- CN-A- 102 883 753
- CN-A- 105 188 791
- CN-A- 106 924 822
- US-A1- 2007 010 632
- US-A1- 2007 010 632

## Description

The embodiments relate to the field of implantable medical devices, in particular to an absorbable iron-based implantable device.

Currently, absorbable implantable medical materials mainly include degradable polymers and absorbable metallic materials. Absorbable metallic materials include magnesium-based alloys, iron-based alloys, zinc-based alloys, pure iron, and the like. Compared with other absorbable materials, the iron-based alloy and the pure iron have excellent mechanical properties comparable to those of permanently implantable medical metallic materials such as stainless steel, cobalt-chromium alloy and platinum-chromium alloy and the like, thus it is possible to use the iron-based alloy and the pure iron to produce an implantable medical device with smaller volume but better mechanical properties, so as to guarantee better clinical operability and effectiveness. An ideal design idea of the absorbable medical device is that the absorbable medical device can provide adequate support in the recovery period of the lesion site, and after healing of the lesion, the absorbable implantable medical device will be corroded or degraded as soon as possible and be absorbed by the body. However, for a long time, an outstanding problem of absorbable medical devices made of iron-based alloy and pure iron that has not yet been solved is that the main iron corrosion products are insoluble in body fluid, although the solid corrosion products can be absorbed and metabolized by the flow of intercellular fluid and engulfment of macrophages, but it takes a long time, resulting in a large amount of corrosion products retained in the tissue for a long time, and possibly a long-term biological risk.

The results showed that after implantation into a body, the iron-based stent was first corroded to generate Fe²⁺, which was easily oxidized to Fe³⁺ in an aerobic environment. There are both PO₄³⁻ and OH- ions in tissue fluid, and thermodynamically, the generated Fe²⁺ will preferably bind to PO₄³⁻ ions to form ferrous phosphate Fe₃(PO₄)₂ precipitate which is more insoluble, and then bind to OH- ions to form Fe(OH)₂ precipitate until complete consumption of phosphate ions in the local tissue, the Fe(OH)₂ precipitate will be further oxidized to ferric hydroxide Fe(OH)₃ precipitate. Thermodynamically, the generated Fe³⁺ ions will preferably react with hydroxide ions to generate Fe(OH)₃ precipitate, due to the fact that iron phosphate is stable only at pH of less than 5, and generally the pH of body fluid is more than 5, so that Fe³⁺ and PO₄³⁻ ions are difficult to form iron phosphate precipitate in vivo. After Fe²⁺ ions generated by corrosion of the iron-based stent completely consume phosphate radicals in the local tissue fluid at the implanted site, it is necessary for phosphate radicals in the tissue fluid in other sites to diffuse to the implanted site to supply, and the diffusion process will take time. In contrast, while the concentration of OH- ions is lower than that of phosphate ions in the local tissue at the implanted site, OH- ions can be rapidly supplied by water dissociation in situ, thus it is more favorable to form ferrous hydroxide kinetically.

Therefore, most of the solid corrosion products after the iron-based stent being implanted into the body are iron hydroxides and iron oxides formed after subsequent dehydration, and a very small amount of corrosion product is ferrous phosphate. In the environment of pH = 7.4 and the temperature being 37 °C (in vivo), the solubility products of Fe(OH)₂ and Fe(OH)₃ are 7.19 × 10⁻¹⁷ and 1.22 × 10⁻³⁸ respectively, i.e., in this environment, the concentration of free Fe²⁺ when the Fe(OH)₂ precipitate reaches the dissolution equilibrium is about 60 mg/L, while the concentration of free Fe³⁺ when the Fe(OH)₃ precipitate reaches the dissolution equilibrium is a dozen orders of magnitude lower than that of Fe²⁺, about 4.3 × 10⁻¹⁴ mg/L, closing to zero. Therefore, when Fe²⁺ generated by corrosion of the iron-based stent is oxidized to Fe³⁺ in the process of diffusing to the peripheral oxygen-enriched area, a Fe(OH)₃ solid corrosion product is easily and quickly formed locally, and due to a very low solubility product thereof, a free Fe³⁺ concentration after reaching the dissolution equilibrium is very low, subsequently it is hard to gradually dissolve and metabolize the Fe(OH)₃ corrosion product by new iron ions ionized by movement of the dissolution equilibrium of Fe(OH)₃ to the right after Fe³⁺ being diffused and absorbed and metabolized by the tissue. The only way for the body to remove Fe(OH)₃, an insoluble solid corrosion product, is through phagocytosis and transportation of phagocytes, such as macrophages. In addition, if the Fe(OH)₃ solid corrosion products are agglomerated together, phagocytes can only contact the outermost layer of the corrosion products, leading to a lower efficiency on phagocytizing and transporting the solid corrosion products. Thus, the removal rate of Fe(OH)₃ solid corrosion products will become slower, resulting in a longer absorption period.

For the problem that the iron corrosion products are difficult to absorb in vivo and need a long absorption period, the prior art adopts a method of adding an antioxidant to promote the generation of the soluble iron corrosion product, the antioxidant being, for example, water-soluble polyphenol, tocopherol, sodium tripolyphosphate and the like, however, the above mentioned antioxidants has the following disadvantages: 1. the antioxidant has a poor stability, and the chemical property thereof is easy to change under the conditions of illumination, oxygen enrichment or specific pH, leading to a loss of the antioxidation effect; 2. the water-soluble antioxidant is dissolved in blood and water-based tissue fluid both in the transportation process and after implantation to lose a large amount itself, and the liposoluble antioxidant is too difficult to dissolve in the water-soluble tissue fluid, leading to a poor anti-oxidation effect, thus it is difficult to promote the generation of the iron corrosion product Fe(OH)₂ having a higher solubility product; 3. most of these antioxidants have a fast release rate initially, while iron-based alloy implantable medical devices usually start to corrode in large in 3 months or so in order to ensure the stability of mechanical properties at the earlier stage. Therefore, the antioxidants have already completely been consumed while the iron substrates haven't subjected corrosion yet, and have no effect on promoting the iron absorption.

US 2007/010632 A discloses a medical device having at least one surface coated with a polymer comprising monomeric units which have been functionalized with antioxidants. The polymer is formed by coupling the antioxidants to monomeric units and subsequently enzymatically polymerizing the functionalized monomeric units.

WO 2008/034031 A shows an endoprosthesis, e.g. a stent, having a member comprising a bioerodible material and an antioxidant carried by said member. The antioxidant can be provided within a matrix or carrier material which in some embodiments is a metal or a polymer. The bioerodible material can be iron or magnesium.

In consideration of this, it is necessary to provide an absorbable iron-based implantable device capable of promoting a long-acting iron absorption.

The object of the invention is solved by an absorbable iron-based implantable device according to claim 1.

The absorbable iron-based implantable device includes an iron-based substrate and an iron absorption promoter that is attached to the iron-based substrate, where the iron absorption promoter is a polymer containing an antioxidant structural unit on the molecular chain thereof.

The iron absorption promoter is capable of being degraded to release an antioxidant.

In one embodiment, the antioxidant is at least one selected from ascorbic acid, procyanidin, vitamin E, glutathione, and lipoic acid.

In one embodiment, the weight-average molecular weight of the iron absorption promoter is 1,000-500,000.

In one embodiment, the iron absorption promoter is a procyanidin polymer.

In one embodiment, the iron absorption promoter further includes at least one degradable polymeric structural unit.

In one embodiment, the iron absorption promoter further includes at least one non-degradable polymeric structural unit.

In one embodiment, the degradable polymer is at least one selected from degradable polyester, polyamino acid, polyanhydride and glucan.

In one embodiment, the degradable polyester is at least one selected from polylactic acid, polyglycolic acid, polysuccinate, poly(β-hydroxybutyrate), polycaprolactone, and polyethylene adipate.

In one embodiment, polyamino acid is at least one selected from polylysine, polyaspartic acid, polycysteine, polymethionine, polyornithine, and polyglutamic acid.

In one embodiment, the polyanhydride is at least one selected from polyoxalate anhydride, polybutanedioic anhydride, polyadipic anhydride, polysebacic anhydride, polydodecanoic anhydride, polycitric anhydride, polymalic anhydride, polysuccinic anhydride, polytartaric anhydride, polyitaconic anhydride, and polymaleic anhydride.

In one embodiment, the non-degradable polymer is at least one selected from polyethylene glycol, polyacrylate, polysuccinate, polycarbonate, polyethylene vinyl acetate, poly(n-butyl methacrylate), polystyrene, polyvinylidene fluoride, polyvinyl pyrrolidone, and polyhexafluoroethylene.

The mass ratio of the iron absorption promoter to the iron-based substrate is 0.05:1 to 3:1.

In one embodiment, the mass ratio of the iron absorption promoter to the iron-based substrate is 0.15:1 to 1:1.

In one embodiment, the iron absorption promoter is attached to the surface of the iron-based substrate; alternatively, the iron absorption promoter is filled inside of the iron-based substrate.

In one embodiment, the absorbable iron-based implantable device is a cardiovascular stent, a cerebrovascular stent, a peripheral vascular stent, a biliary stent, an esophageal stent, an airway stent, an occluding device, or an orthopedic implant.

In one embodiment, the absorbable iron-based implantable device further includes an active drug attached to the iron-based substrate which is at least one selected from antiangiogenic drugs, antiplatelet drugs, antithrombotic drugs, anti-inflammatory drugs, and anti-sensitizing drugs.

An iron absorption promoter is attached to the iron-based substrate of the absorbable iron-based implantable device described above, the iron absorption promoter is a polymer containing an antioxidant structural unit, and after the absorbable iron-based implantable device is implanted into a body, the antioxidant is gradually released as the iron absorption promoter is gradually degraded or broken, and may promote the iron absorption.

The foregoing objects, features and advantages of the embodiments will become more apparent from the following detailed description. In the following, numerous details are set forth to describe the embodiments. However, the embodiments may be implemented by many other manners different than those described herein and similar modifications may be made by those of ordinary skill in the art, thus the embodiments should not be construed as limiting.

Unless defined otherwise, all technical and scientific terms used herein have the same meaning as commonly understood by one of ordinary skill in the art to which the present disclosure belongs. The terms used herein in the description of the present disclosure are for the purpose of describing particular embodiments only and not intended to be limiting of the present disclosure.

In one embodiment, an absorbable iron-based implantable device includes an iron-based substrate and an iron absorption promoter attached to the iron-based substrate, the iron absorption promoter being a polymer containing an antioxidant structural unit on the molecular chain thereof, and being capable of being degraded to release the antioxidant.

The iron-based substrate may be a pure iron substrate or an iron-based alloy substrate. For example, the material of iron-based alloy substrate is an iron-based alloy having a carbon content of not higher than 2.11 wt.%.

The iron absorption promoter may be attached to the surface of the iron-based substrate in a film forming manner. For example, the iron absorption promoter is attached to the outer surface, the inner surface or the side of the iron-based substrate. Alternatively, the iron absorption promoter is attached to the outer surface, the inner surface and the side surface of the iron-based substrate at the same time. Or the iron absorption promoter is attached to the outer and inner surfaces of the iron-based substrate, etc. That is, the position of the film formed by the iron absorption promoter on the iron-based substrate is not limited. Also, the distribution of the iron absorption promoter on the surface of the iron-based substrate may be either uniform or non-uniform.

In a further embodiment, the iron absorption promoter may be filled inside of the iron-based substrate. Gaps, pores, grooves, through-holes or inner cavities and the like can be formed on the iron-based substrate, so that the iron absorption promoter is filled into the corresponding gaps, pores, grooves, through-holes or inner cavities, so that the iron absorption promoter is filled inside of the iron-based substrate.

For example, the antioxidant is at least one selected from ascorbic acid, procyanidine, vitamin E, glutathione and lipoic acid. An antioxidant is polymerized to form an iron absorption promoter through a polymerization reaction, an antioxidant structural unit being a repeating unit on the molecular chain of the iron absorption promoter, and the antioxidant structural units being connected through chemical bonds. The antioxidant is released as the iron absorption promoter is degraded or broken.

The antioxidant released from the iron absorption promoter is at least one selected from ascorbic acid, procyanidin, vitamin E, glutathione and lipoic acid. The antioxidant released from the iron absorption promoter is a small molecule in the normal metabolic process of human tissue cells or a daily nutrient which can be safely untaken, such that the degraded or released product of the iron absorption promoter in vivo has a good biocompatibility with a human body, and harmful substance to the human body is not generated.

The antioxidant released during degradation of the above iron absorption promoter is an antioxidant showing a strong reduction effect, and when the iron absorption promoter is degraded or broken in vivo to release the antioxidant, oxygen in situ and near the absorbable iron-based implantable device can be consumed, to ensure that Fe²⁺ generated by corrosion is not immediately oxidized into Fe³⁺, but exists in the form of Fe²⁺ and precipitate thereof having a higher solubility, thereby facilitating diffusion and absorption of the iron corrosion product. Moreover, the iron absorption promoter can delay the conversion of Fe²⁺ to Fe³⁺, and free Fe²⁺ can diffuse out along with tissue fluid. The farther away from the iron-based substrate, the higher the concentration of oxygen ions in the tissue, until the level of oxygen concentration in normal tissues is recovered. The diffused Fe²⁺ ions are continuously oxidized to Fe³⁺ ions in oxygen-enriched tissues far away from the iron-based substrate, the Fe³⁺ ions are precipitated into dispersive Fe(OH)₃ solid corrosion products in surrounding tissues in which the iron-based substrate is implanted in situ. The more dispersive the Fe(OH)₃ solid corrosion product is, the more beneficial to improve the efficiency of phagocytosis and transportation and metabolism of ferric corrosion products by phagocytic cells, so that the absorption period of the absorbable iron-based implantable device in vivo is shortened.

In addition, in a physiological environment, after the absorbable iron-based implantable device is implanted into a body, the antioxidant is gradually released as the iron absorption promoter is gradually degraded or broken, so that the release rate of the antioxidant is delayed, the utilization rate of the antioxidant is improved, and the effect for promoting the long-acting iron absorption is achieved.

In a simulated physiological environment, such as in a saline solution, the iron absorption promoter may also be gradually degraded or broken to gradually release the antioxidant.

It can be appreciated that the antioxidant forming the iron absorption promoter may be any other antioxidant that shows antioxidant capacity and good biocompatibility with the human body.

If the molecular weight of the iron absorption promoter is too low, the release rate of the antioxidant will be too fast to facilitate improving the long-acting iron absorption promoting effect thereof, while if the molecular weight of the iron absorption promoter is too high, it will make the synthesis difficult and mechanical properties too poor to coat on the surface of the iron-based substrate. Thus, the weight-average molecular weight of the iron absorption promoter is from 1,000 to 500,000. For example, the weight-average molecular weight of the iron absorption promoter is from 3,000 to 300,000.

Meanwhile, the above iron absorption promoter is a polymer containing antioxidant structural units and has the advantage of high stability comparing to most natural antioxidants. Under illumination, oxygen enrichment or a specific pH environment, the chemical property of the above iron absorption promoter is difficult to change, enabling a long-acting and stable existence. In addition, the iron absorption promoter has a poor solubility in water. Therefore, the iron absorption promoter can exist stably in a body, and after the absorbable iron-based implantable device is implanted into the body, the iron absorption promoter is difficult to dissolve in aqueous tissue fluid, resulting in a little lose and thereby promoting the absorption of iron in long term.

Moreover, the iron absorption promoter has good stability and low environmental requirement on product storage, so that the product failure caused by the failure of the iron absorption promoter during storage is avoided, and the shelf life is prolonged.

In one embodiment, the iron absorption promoter further includes at least one degradable polymeric structural unit. The iron absorption promoter is formed by polymerization of a degradable polymer monomer and the above antioxidant. On the molecular chain of the iron absorption promoter, the degradable polymer structural unit is connected with the antioxidant structural unit through a chemical bond. Here, the degradable polymer structural unit refers to the repeating unit in the degradable polymer molecular chain.

In one embodiment, the degradable polymer is selected from at least one forming degradable polyester, polyamino acid, polyanhydride and glucan.

In one embodiment, the degradable polyester is at least one selected from polylactic acid, polyglycolic acid, polysuccinate, poly(β-hydroxybutyrate), polycaprolactone, and polyethylene adipate.

In one embodiment, polyamino acid is at least one selected from polylysine, polyaspartic acid, polycysteine, polymethionine, polyornithine, and polyglutamic acid.

In one embodiment, the polyanhydride is at least one selected from polyoxalate anhydride, polybutanedioic anhydride, polyadipic anhydride, polysebacic anhydride, polydodecanoic anhydride, polycitric anhydride, polymalic anhydride, polysuccinic anhydride, polytartaric anhydride, polyitaconic anhydride, and polymaleic anhydride.

Polymerization of the degradable polymer monomer and the antioxidant facilitates improving the stability of the antioxidant, further delaying the release rate of the antioxidant, such that after the absorbable iron-based implantable device is implanted into a body, the antioxidant is gradually and slowly released to keep consistent with the degradation period of the iron-based substrate as far as possible, enabling a long-acting effect on promoting the iron absorption to a maximum level, and the chain segment formed by the degradable polymer is gradually degraded and absorbed as the antioxidant is released.

It can be appreciated that in other embodiments, in addition to the degradable polymer monomers listed above, other degradable polymer monomers capable of polymerizing with an antioxidant to form an iron absorption promoter and having good biocompatibility with the human body can also be used in the absorbable iron-based implantable device to promote a long-lasting absorption of iron.

In a further embodiment, the iron absorption promoter further includes at least one non-degradable polymeric structural unit. The non-degradable polymeric structural unit is a repeating unit on the non-degradable polymer molecular chain. The iron absorption promoter is formed by polymerization of a non-degradable polymer monomer with an antioxidant. In one embodiment, the non-degradable polymer is at least one selected from polyethylene glycol, polyacrylate, polysuccinate, polycarbonate, polyethylene vinyl acetate, poly(n-butyl methacrylate), polystyrene, polyvinylidene fluoride, polyvinyl pyrrolidone, and polyhexafluoroethylene.

In one embodiment, the iron absorption promoter further includes at least one degradable polymeric structural unit and at least one non-degradable polymeric structural unit. For example, the varieties of degradable and non-degradable polymers are the same as the varieties of degradable and non-degradable polymers listed above, respectively.

The addition of the non-degradable polymeric structural unit may improve hydrophilicity or hydrophobicity, or mechanical properties of the iron absorption promoter, or stability and the release rate of the antioxidant.

It can be appreciated that in other embodiments, in addition to the non-degradable polymeric monomers listed above, other non-degradable polymeric monomers which are capable of polymerizing with an antioxidant or an antioxidant together with a degradable polymeric monomer to form an iron absorption promoter and are biocompatible with the human body, may also be used in the absorbable iron-based implantable devices described above to improve hydrophilicity or hydrophobicity, or mechanical properties of the iron absorption promoter, or stability and the release rate of the antioxidant.

For example, the antioxidant is copolymerized with the degradable polymer monomer and/or the non-degradable polymer monomer by random copolymerization, alternating copolymerization, block copolymerization or graft copolymerization. The iron absorption promoter formed by polymerization can be gradually release the antioxidant.

The lower mass of the iron absorption promoter has a novel effect on promoting the absorption of iron. The higher mass of the iron absorption promoter has a good effect on promoting the absorption of iron, but due to high mass and large volume thereof, physical properties of the absorbable iron-based implantable device are seriously affected. Therefore, the mass ratio of the iron absorption promoter to iron in the iron-based substrate is 0.05:1 to 3:1 in combination with the iron absorption effect and physical properties. In one embodiment, the mass ratio of the iron absorption promoter to iron in the iron-based substrate is 0.15:1 to 1:1.

In one embodiment, the absorbable iron-based implantable device further includes an active drug. The active drug may be coated directly on the iron-based substrate, or filled on the iron-based substrate, or dispersed in a film formed from the iron absorption promoter, or dispersed in the degradable polymer layer.

The active drug is at least one selected from antiangiogenic drugs, antiplatelet drugs, antithrombotic drugs, anti-inflammatory drugs, and anti-sensitizing drugs.

In particular, the antiangiogenic drug is at least one selected from taxol, rapamycin and rapamycin derivatives. The antiplatelet drug is cilostazol. The antithrombotic drug is heparin. The anti-inflammatory drug is dexamethasone. The anti-sensitizing drug is at least one selected from calcium gluconate, chlorpheniramine and cortisone.

An iron absorption promoter is attached to the iron-based substrate of the absorbable iron-based implantable device described above, the iron absorption promoter being a polymer containing an antioxidant structural unit. After the absorbable iron-based implantable device is implanted into a body, as the absorbable iron-based implantable device is gradually and slowly degraded, an antioxidant is gradually released, thereby enabling a long-acting effect and effectively delaying the release rate of the antioxidant, and the antioxidant which is released by the iron absorption promoter is interacted with an iron corrosion product more fully during the gradual corrosion process of the iron-based substrate, thereby promoting the absorption of the iron corrosion product.

The absorbable iron-based implantable device is a cardiovascular stent, a cerebrovascular stent, a peripheral vascular stent, a biliary tract stent, an esophageal stent, an airway stent, an occluding device, an orthopaedic implant and the like.

For preparation of the above-mentioned absorbable iron-based implantable device, first an iron-based substrate is prepared, and then an iron absorption promoter and/or a drug-containing degradable polymer layer and the like are formed on the iron-based substrate. For example, a lumen iron-based substrate may be formed by laser cutting, and then an iron absorption promoter and/or drug-containing degradable polymer layer may be formed on the iron-based substrate by plasma spraying, ultrasonic atomization spraying, and the like.

The above-mentioned iron absorption promoter is commercially available or is obtained by polymerization using a polymerization method. For instance, a melt polymerization process is used to copolymerize an antioxidant and a polymer monomer to form an iron absorption promoter, for example, the melt polymerization process is used to form a polylactic acid vitamin E copolymer. Alternatively, a ring-opening polymerization is used to form a copolymer of an antioxidant and a polymer monomer, for example, ascorbic acid is used as an initiator to initiate ring-opening polymerization of caprolactone to form ascorbic acid polycaprolactone copolymer. As another example, vitamin C (ascorbic acid), anhydrous potassium carbonate, and N, N-dimethylformamide are dissolved and mixed to react to form a benzyl-protected vitamin C precursor. After the benzyl protected vitamin C precursor is dissolved in dichloromethane, the solution of acrylamide is slowly added dropwise to form an acrylate-modified benzyl-protected vitamin C monomer. A polylactic acid homopolymer is formed by ring-opening polymerization of L-type lactide. The homopolymer and the vitamin C precursor are synthesized under the condition of copper bromide and anhydrous toluene to form a block compolyer which is then debenzylated by hydrogen to obtain polylactic acid-block-polyascorbic acid acrylate copolymer.

The absorbable iron-based implantable device described above are further illustrated by the following examples.

The following characterization and test methods are employed in the following embodiments:

### 1. Characterization of the effect of promoting the iron absorption

Taking an iron-based alloy vascular stent containing an iron absorption promoter as an example, a test that an iron-based alloy stent containing the iron absorption promoter was implanted into a healthy mini porcine coronary artery model was performed to detect the absorption/metabolism percentage of iron in a blood vessel where the iron-based alloy vascular stent was positioned, and the effect of promoting the iron absorption by the iron absorption promoter in vivo is evaluated by the absorption/metabolism percentage of iron. The method was as follows: measuring the mass of iron in the implanted iron-based alloy vascular stent as M₀, then implanting iron-based alloy vascular stent containing the iron absorption promoter in the same batch and same specification into a porcine coronary artery, taking out the iron-based alloy vascular stent and a blood vessel segment where the iron-based alloy vascular stent was positioned after 36 months, carefully trimming redundant matters such as adipose tissue and myocardial tissue surrounding the outer membrane of the blood vessel to ensure the length of the blood vessel equivalent to that of the iron-based alloy vascular stent implanted therein, measuring the iron content in the iron-based alloy vascular stent as well as the blood vessel segment where the iron-based alloy vascular stent was positioned as M₁, and the iron ion content in the blank blood vessel was too low and can be ignored herein. Thus, during the implantation period, the iron absorption/metabolism percentage was W, W = (M₀ - M₁)/M₀*100%.

Here, the method for testing the iron content was as follows: digesting the stent or the stent together with the blood vessel in a microwave digestion instrument by using concentrated nitric acid, then testing the concentration of iron ion in the solution by using an Agilent 240FS atomic absorption spectrometer under the conditions of a wavelength of 248.3 nm, a slit of 0.2 nm, acetylene as combustion-supporting gas and a flow rate of 2.0 L/min, and further calculating the mass of iron in the sample.

The better the effect of promoting iron absorption by the iron absorption promoter, the higher the percentage of iron absorption/metabolism. For an iron-based alloy implantable device without an antioxidant or an iron-based alloy implantable device containing a small molecular antioxidant, the iron absorption/metabolism percentage was generally within 15% after implantation into a porcine coronary artery for 36 months, while after adding a high molecular iron absorption promoter, the iron absorption/metabolism percentage was more than 15%, and the most desirable effect of promoting iron absorption was that the iron absorption/metabolism percentage equaled to 100%.

### 2. The method for detecting the weight-average molecular weight of the polymer:

The weight-average molecular weight of the polymer was detected using a GPC-multi-angle light scattering detector from Wyatt, USA, combined with a molecular weight test system. The test system included a liquid phase pump and a sample injector from Agilent, USA, an Agilent PL MIXED-C type GPC column (size: 7.5 × 300mm, 5 microns) from Agilent, USA, and a multi-angle light scattering detector and a differential detector from Wyatt, USA. the detection condition was as follows: fluid phase: tetrahydrofuran; pump rate: 1 mL/min; loading volume of sample: 100 µL; laser wavelength: 663.9 nm; test temperature: 35 °C.

### Embodiment 1

Proanthocyanidin polymer having a weight-average molecular weight of 1000 was dissolved in ethyl acetate to formulate a solution having the concentration of the proanthocyanidin polymer of 10 mg/mL, the formulated solution was then uniformly sprayed on the inner surface and the outer surface of an iron-based alloy coronary stent substrate having a nominal diameter of 4.0 mm and a wall thickness of 60 µm to form an iron absorption promoter coating having a thickness of 5 µm, thus obtaining an iron-based alloy coronary stent. Here, the mass ratio of the iron absorption promoter to iron in the iron-based alloy coronary stent substrate was 0.05:1. A polylactic acid (having a weight-average molecular weight of 20,000) rapamycin-loaded coating having a thickness of 3 µms was continuously sprayed on the surface of the iron absorption promoter coating to obtain the iron-based alloy coronary stent. The prepared iron-based alloy coronary stent was implanted into a mini porcine coronary artery, maintaining an overdilation ratio in the range of 1.1:1 to 1.2:1 in the implantation process. A follow-up was performed 36 months after implantation, the remaining iron-based alloy coronary stent and surrounding vascular tissue thereof were removed, and the percentage of iron metabolized by the tissue tested as described above was 20%.

### Embodiment 2

Tocopherol polyethylene glycol maleate having a weight-average molecular weight of 5000 was dissolved in ethyl acetate to formulate a solution having the concentration of tocopherol polyethylene glycol maleate of 10 mg/mL, the formulated solution was then uniformly sprayed on the inner surface and the outer surface of an iron-based alloy coronary stent substrate having a nominal diameter of 3.0 mm and a wall thickness of 50 µm to form an iron absorption promoter coating having a thickness of 10 µm, thus obtaining an iron-based alloy coronary stent. Here, the mass ratio of the iron absorption promoter to iron in the iron-based alloy coronary stent substrate was 0.15:1. The prepared iron-based alloy coronary stent was implanted into a mini porcine coronary artery, maintaining an overdilation ratio in the range of 1.1:1 to 1.2:1 in the implantation process. A follow-up was performed 36 months after implantation, the remaining iron-based alloy coronary stent and surrounding vascular tissue thereof were removed, and the percentage of iron metabolized by the tissue tested as described above was 26%.

### Embodiment 3

Polylactic acid block polycysteine coupled glutathione having a weight-average molecular weight of 10,000 was dissolved in ethyl acetate to formulate a solution having a concentration of 5 mg/mL, the formulated solution was then uniformly sprayed on the inner surface and the outer surface of an iron-based alloy coronary stent substrate having a nominal diameter of 3 mm and a wall thickness of 50 µm to form an iron absorption promoter coating having a thickness of 15 µm, thus obtaining an iron-based alloy coronary stent. Here, the mass ratio of the iron absorption promoter to iron in the iron-based alloy coronary stent substrate was 0.25:1. The prepared iron-based alloy coronary stent was implanted into a mini porcine coronary artery, maintaining an overspread ratio in the range of 1.1:1 to 1.2:1 in the implantation process. A follow-up was performed 36 months after implantation, the remaining iron-based alloy coronary stent and surrounding vascular tissue thereof were removed, and the percentage of iron metabolized by the tissue tested as described above was 30%.

### Embodiment 4

Polylactic acid vitamin E copolymer having a weight-average molecular weight of 50,000 was dissolved in dichloromethane to formulate a solution having the concentration of 5 mg/mL, the formulated solution was then uniformly sprayed on the inner surface and the outer surface of an iron-based alloy coronary stent substrate having a nominal diameter of 3.0 mm and a wall thickness of 50 µm to form an iron absorption promoter coating having a thickness of 15 µm, a polylactic acid (having a weight-average molecular weight of 20,000) paclitaxel-loaded drug layer having a thickness of 3 µm was continuously sprayed on the surface of the iron absorption promoter coating to obtain an iron-based alloy coronary stent. Here, the mass ratio of the iron absorption promoter to iron in the iron-based alloy coronary stent substrate was 0.25:1. The prepared iron-based alloy coronary stent was implanted into a mini porcine coronary artery, maintaining an overdilation ratio in the range of 1.1:1 to 1.2:1 in the implantation process. A follow-up was performed 36 months after implantation, the remaining iron-based alloy coronary stent and surrounding vascular tissue thereof were removed, and the percentage of iron metabolized by the tissue tested as described above was 35%.

### Embodiment 5

Lipoic acid polylysine polyethylene glycol copolymer having a weight-average molecular weight of 100,000 was dissolved in ethyl acetate to formulate a solution having the concentration of 10 mg/mL, the formulated solution was then uniformly sprayed on the inner surface and the outer surface of an iron-based alloy coronary stent substrate having a nominal diameter of 3.0 mm and a wall thickness of 50 µm to form an iron absorption promoter coating having a thickness of 25 µm, thus obtaining an iron-based alloy coronary stent. Here, the mass ratio of the iron absorption promoter to iron in the iron-based alloy coronary stent substrate was 0.5:1. The prepared iron-based alloy coronary stent was implanted into a mini porcine coronary artery, maintaining an overdilation ratio in the range of 1.1:1 to 1.2:1 in the implantation process. A follow-up was performed 36 months after implantation, the remaining iron-based alloy coronary stent and surrounding vascular tissue thereof were removed, and the percentage of iron metabolized by the tissue tested as described above was 40%.

### Embodiment 6

Ascorbic acid polycaprolactone copolymer having a weight-average molecular weight of 300,000, polylactic acid having a weight-average molecular weight of 100,000 and dexamethasone were dissolved in ethyl acetate based on a blending ratio of 1:1:1 by mass to formulate a solution having a total concentration of 6 mg/mL, the formulated solution was then uniformly sprayed on the inner surface and the outer surface of an iron-based alloy coronary stent substrate having a nominal diameter of 3.0 mm and a wall thickness of 50 µm to form a blended coating containing an iron absorption promoter with a thickness of 25 µm, thus obtaining an iron-based alloy coronary stent. Here, the mass ratio of the iron absorption promoter to iron in the iron-based alloy coronary stent substrate was 0.5:1. The prepared iron-based alloy coronary stent was implanted into a mini porcine coronary artery, maintaining an overdilation ratio in the range of 1.1:1 to 1.2:1 in the implantation process. A follow-up was performed 36 months after implantation, the remaining iron-based alloy coronary stent and surrounding vascular tissue thereof were removed, and the percentage of iron metabolized by the tissue tested as described above was 45%.

### Embodiment 7

Polylactic acid block polyascorbic acid acrylate having a weight-average molecular weight of 500,000 was dissolved into trichloromethane to formulate a solution having a concentration of 1.5 mg/mL, the formulated solution was then uniformly sprayed on the inner surface and the outer surface of an iron-based alloy coronary stent substrate having a nominal diameter of 2.5 mm and a wall thickness of 40 µm to form an iron absorption promoter coating having a thickness of 33 µm, thus obtaining an iron-based alloy coronary stent. Here, the mass ratio of the iron absorption promoter to iron in the iron-based alloy coronary stent substrate was 1:1. The prepared iron-based alloy coronary stent was implanted into a mini porcine coronary artery, maintaining an overdilation ratio in the range of 1.1:1 to 1.2:1 in the implantation process. A follow-up was performed 36 months after implantation, the remaining iron-based alloy coronary stent and surrounding vascular tissue thereof were removed, and the percentage of iron metabolized by the tissue tested as described above was 55%.

### Embodiment 8

Lipoic acid glucan copolymer having a weight-average molecular weight of 500,000 was dissolved in ethyl acetate to formulate a solution having the concentration of the lipoic acid glucan copolymer of 2 mg/mL, the formulated solution was then uniformly sprayed on the inner surface and the outer surface of an iron-based alloy coronary stent substrate having a nominal diameter of 2.5 mm and a wall thickness of 40 µm to form an iron absorption promoter coating having a thickness of 50 µm, thus obtaining an iron-based alloy coronary stent. Here, the mass ratio of the iron absorption promoter to iron in the iron-based alloy coronary stent substrate was 2:1. The prepared iron-based alloy coronary stent was implanted into a mini porcine coronary artery, maintaining an overdilation ratio in the range of 1.1:1 to 1.2:1 in the implantation process. A follow-up was performed 36 months after implantation, the remaining iron-based alloy coronary stent and surrounding vascular tissue thereof were removed, and the percentage of iron metabolized by the tissue tested as described above was 60%.

### Embodiment 9

Lipoic acid polyaspartic acid polyethylene glycol copolymer having a weight-average molecular weight of 100,000 (the preparation method is performed according to the method disclosed in CN1055242722 A) is dissolved in ethyl acetate to formulate a solution having the concentration of 10 mg/mL, the formulated solution was then uniformly sprayed on the inner surface and the outer surface of an iron-based alloy coronary stent substrate having a nominal diameter of 2.5 mm and a wall thickness of 30 µm to form an iron absorption promoter coating having a thickness of 50 µm, thus obtaining an iron-based alloy coronary stent. Here, the mass ratio of the iron absorption promoter to iron in the iron-based alloy coronary stent substrate was 3:1. The prepared iron-based alloy coronary stent was implanted into a mini porcine coronary artery, maintaining an overdilation ratio in the range of 1.1:1 to 1.2:1 in the implantation process. A follow-up was performed 36 months after implantation, the remaining iron-based alloy coronary stent and surrounding vascular tissue thereof were removed, and the percentage of iron metabolized by the tissue tested as described above was 60%.

### Comparative Example 1

Proanthocyanidin having a molecular weight of 594 was dissolved in ethanol to formulate a solution having a concentration of the proanthocyanidin of 10 mg/mL, the formulated solution was then uniformly sprayed on the inner surface and the outer surface of an iron-based alloy coronary stent substrate having a nominal diameter of 4.0 mm and a wall thickness of 60 µm to form an iron absorption promoter coating having a thickness of 5 µm, thus obtaining an iron-based alloy coronary stent. Here, the mass ratio of proanthocyanidin to iron in the iron-based alloy coronary stent substrate was 0.05:1. The prepared iron-based alloy coronary stent was implanted into a mini porcine coronary artery, maintaining an overdilation ratio in the range of 1.1:1 to 1.2:1 in the implantation process. A follow-up was performed 36 months after implantation, the remaining iron-based alloy coronary stent and surrounding vascular tissue thereof were removed, and the percentage of iron metabolized by the tissue tested as described above was 10%.

### Comparative Example 2

Vitamin C was dissolved in purified water to formulate a solution having a concentration of vitamin C of 10 mg/mL, the formulated solution was then uniformly sprayed on the inner surface and the outer surface of an iron-based alloy coronary stent substrate having a nominal diameter of 2.5 mm and a wall thickness of 40 µm to form an iron absorption promoter coating having a thickness of 33 µm, thus obtaining an iron-based alloy coronary stent. Here, the mass ratio of vitamin C to iron in the iron-based alloy coronary stent substrate was 1:1. The prepared iron-based alloy coronary stent was implanted into a mini porcine coronary artery, maintaining an overdilation ratio in the range of 1.1:1 to 1.2:1 in the implantation process. A follow-up was performed 36 months after implantation, the remaining iron-based alloy coronary stent and surrounding vascular tissue thereof were removed and the percentage of iron metabolized by the tissue tested as described above was 15%.

It can be seen that after implantation of the iron-based alloy coronary stents of Embodiments 1-9, the percentage of iron metabolized by the tissue is higher than that of Comparative Example 1 and Comparative Example 2, indicating that the iron absorption promoter of Embodiments 1-9 does promote a long-acting iron absorption.

The various features of the above-mentioned embodiments may be combined in any way, and in order to simplify the description, not all possible combinations of the features of the above-mentioned embodiments are described, however, as long as there is no conflict between these features, they should be considered to be within the scope of the description.

## Claims

1. An absorbable iron-based implantable device, comprising:
an iron-based substrate and an iron absorption promoter attached to the iron-based substrate,
wherein the iron absorption promoter is a polymer, and an antioxidant structural unit is contained in the molecular chain of the iron absorption promoter,
wherein the iron absorption promoter is capable of being degraded to release an antioxidant, and
wherein the mass ratio of the iron absorption promoter to iron in the iron-based substrate is 0.05:1 to 3:1.

2. The absorbable iron-based implantable device of claim 1, wherein the antioxidant is at least one selected from the group consisting of: ascorbic acid, procyanidin, vitamin E, glutathione, and lipoic acid.

3. The absorbable iron-based implantable device of claim 1, wherein the iron absorption promoter has a weight-average molecular weight of 1,000 to 500,000.

4. The absorbable iron-based implantable device of claim 1, wherein the iron absorption promoter is a procyanidin polymer.

5. The absorbable iron-based implantable device of claim 1, wherein the iron absorption promoter further comprises at least one degradable polymeric structural unit.

6. The absorbable iron-based implantable device of claim 1, wherein the iron absorption promoter further comprises at least one non-degradable polymeric structural unit.

7. The absorbable iron-based implantable device of claim 5, wherein the degradable polymer is at least one selected from the group consisting of: degradable polyester, polyamino acid, polyanhydride, and glucan.

8. The absorbable iron-based implantable device of claim 7, wherein the degradable polyester is at least one selected from the group consisting of: polylactic acid, polyglycolic acid, polysuccinate, poly(β-hydroxybutyrate), polycaprolactone, and polyethylene adipate.

9. The absorbable iron-based implantable device of claim 7, wherein the polyamino acid is at least one selected from the group consisting of: polylysine, polyaspartic acid, polycysteine, polymethionine, polyornithine, and polyglutamic acid.

10. The absorbable iron-based implantable device of claim 7, wherein the polyanhydride is at least one selected from the group consisting of: polyoxalate anhydride, polybutanedioic anhydride, polyadipic anhydride, polysebacic anhydride, polydodecanoic anhydride, polycitric anhydride, polymalic anhydride, polysuccinic anhydride, polytartaric anhydride, polyitaconic anhydride, and polymaleic anhydride.

11. The absorbable implantable device of claim 6, wherein the non-degradable polymer is at least one selected from the group consisting of: polyethylene glycol, polyacrylate, polysuccinate, polycarbonate, polyethylene vinyl acetate, poly(n-butyl methacrylate), polystyrene, polyvinylidene fluoride, polyvinyl pyrrolidone, and polyhexafluoroethylene.

12. The absorbable iron-based implantable device of claim 1, wherein the mass ratio of the iron absorption promoter to iron in the iron-based substrate is 0.15:1 to 1:1.

13. The absorbable iron-based implantable device of claim 1, wherein the iron absorption promoter is attached to a surface of the iron-based substrate or the iron absorption promoter is filled inside of the iron-based substrate.

14. The absorbable iron-based implantable device of claim 1, wherein the absorbable iron-based implantable device is a cardiovascular stent, a cerebrovascular stent, a peripheral vascular stent, a biliary stent, an esophageal stent, an airway stent, an occluding device, or an orthopedic implant.

15. The absorbable iron-based implantable device of claim 1, wherein the absorbable iron-based implantable device further comprises an active drug attached to the iron-based substrate, wherein the active drug is at least one selected from antiangiogenic drugs, antiplatelet drugs, antithrombotic drugs, anti-inflammatory drugs, and anti-sensitizing drugs.

## Patentansprüche

1. Absorbierbare, implantierbare Vorrichtung auf Eisenbasis, die Folgendes umfasst:
ein Substrat auf Eisenbasis und einen Eisenabsorptionspromotor, der an das Substrat auf Eisenbasis gebunden ist,
wobei der Eisenabsorptionspromotor ein Polymer ist und eine Antioxidans-Struktureinheit in der Molekülkette des Eisenabsorptionspromotors enthalten ist,
wobei der Eisenabsorptionspromotor zum Freisetzen eines Antioxidans abgebaut werden kann, und
wobei das Massenverhältnis des Eisenabsorptionspromotors zu Eisen im Substrat auf Eisenbasis 0,05:1 bis 3:1 beträgt.

2. Absorbierbare, implantierbare Vorrichtung auf Eisenbasis nach Anspruch 1, wobei es sich bei dem Antioxidans um mindestens eines handelt, das aus der Gruppe ausgewählt ist, die aus Folgendem besteht: Ascorbinsäure, Procyanidin, Vitamin E, Glutathion und Liponsäure.

3. Absorbierbare, implantierbare Vorrichtung auf Eisenbasis nach Anspruch 1, wobei der Eisenabsorptionspromotor ein gewichtsmittleres Molekulargewicht von 1.000 bis 500.000 hat.

4. Absorbierbare, implantierbare Vorrichtung auf Eisenbasis nach Anspruch 1, wobei der Eisenabsorptionspromotor ein Procyanidin-Polymer ist.

5. Absorbierbare, implantierbare Vorrichtung auf Eisenbasis nach Anspruch 1, wobei der Eisenabsorptionspromotor ferner mindestens eine abbaubare polymere Struktureinheit umfasst.

6. Absorbierbare, implantierbare Vorrichtung auf Eisenbasis nach Anspruch 1, wobei der Eisenabsorptionspromotor ferner mindestens eine nicht abbaubare polymere Struktureinheit umfasst.

7. Absorbierbare, implantierbare Vorrichtung auf Eisenbasis nach Anspruch 5, wobei es sich bei dem abbaubaren Polymer um mindestens eines handelt, das aus der Gruppe ausgewählt ist, die aus Folgendem besteht: abbaubarem Polyester, Polyaminosäure, Polyanhydrid und Glucan.

8. Absorbierbare, implantierbare Vorrichtung auf Eisenbasis nach Anspruch 7, wobei es sich bei dem abbaubaren Polyester um mindestens eines handelt, das aus der Gruppe ausgewählt ist, die aus Folgendem besteht: Polymilchsäure, Polyglykolsäure, Polysuccinat, Poly(β-hydroxybutyrat), Polycaprolacton und Polyethylenadipat.

9. Absorbierbare, implantierbare Vorrichtung auf Eisenbasis nach Anspruch 7, wobei es sich bei der Polyaminosäure um mindestens eine handelt, die aus der Gruppe ausgewählt ist, die aus Folgendem besteht: Polylysin, Polyasparaginsäure, Polycystein, Polymethionin, Polyornithin und Polyglutaminsäure.

10. Absorbierbare, implantierbare Vorrichtung auf Eisenbasis nach Anspruch 7, wobei es sich bei dem Polyanhydrid um mindestens eines handelt, das aus der Gruppe ausgewählt ist, die aus Folgendem besteht: Polyoxalatanhydrid, Polybutandisäureanhydrid, Polyadipinsäureanhydrid, Polysebacinsäureanhydrid, Polydodecansäureanhydrid, Polycitronensäureanhydrid, Polyäpfelsäureanhydrid, Polysuccinsäureanhydrid, Polytartarsäureanhydrid, Polyitaconsäureanhydrid und Polymaleinsäureanhydrid.

11. Absorbierbare, implantierbare Vorrichtung nach Anspruch 6, wobei es sich bei dem nicht abbaubaren Polymer um mindestens eines handelt, das aus der Gruppe ausgewählt ist, die aus Folgendem besteht: Polyethylenglykol, Polyacrylat, Polysuccinat, Polycarbonat, Polyethylenvinylacetat, Poly(n-butylmethacrylat), Polystyrol, Polyvinylidenfluorid, Polyvinylpyrrolidon und Polyhexafluorethylen.

12. Absorbierbare, implantierbare Vorrichtung auf Eisenbasis nach Anspruch 1, wobei das Massenverhältnis des Eisenabsorptionspromotors zu Eisen im Substrat auf Eisenbasis 0,15:1 bis 1:1 beträgt.

13. Absorbierbare, implantierbare Vorrichtung auf Eisenbasis nach Anspruch 1, wobei der Eisenabsorptionspromotor an eine Oberfläche des Substrats auf Eisenbasis gebunden ist oder der Eisenabsorptionspromotor in das Innere des Substrats auf Eisenbasis gefüllt ist.

14. Absorbierbare, implantierbare Vorrichtung auf Eisenbasis nach Anspruch 1, wobei die absorbierbare, implantierbare Vorrichtung auf Eisenbasis ein kardiovaskulärer Stent, ein zerebrovaskulärer Stent, ein peripherer vaskulärer Stent, ein biliärer Stent, ein Ösophagus-Stent, ein Atemwegs-Stent, eine Verschlussvorrichtung oder ein orthopädisches Implantat ist.

15. Absorbierbare, implantierbare Vorrichtung auf Eisenbasis nach Anspruch 1, wobei die absorbierbare, implantierbare Vorrichtung auf Eisenbasis ferner ein aktives Medikament umfasst, das an das Substrat auf Eisenbasis gebunden ist, wobei es sich bei dem aktiven Medikament um mindestens eines handelt, das aus antiangiogenen Medikamenten, plättchenhemmenden Medikamenten, antithrombotischen Medikamenten, entzündungshemmenden Medikamenten und antisensibilisierenden Medikamenten ausgewählt ist.

## Revendications

1. Dispositif implantable absorbable à base de fer, comprenant :
un substrat à base de fer et un promoteur d'absorption de fer fixé au substrat à base de fer,
le promoteur d'absorption de fer étant un polymère, et une unité structurelle antioxydante étant contenue dans la chaîne moléculaire du promoteur d'absorption de fer,
le promoteur d'absorption de fer étant apte à être dégradé pour libérer un antioxydant, et
le rapport de masse entre le promoteur d'absorption de fer et le fer dans le substrat à base de fer étant compris entre 0,05:1 et 3:1.

2. Dispositif implantable absorbable à base de fer selon la revendication 1, l'antioxydant étant au moins l'un des antioxydants choisis dans le groupe constitué par : l'acide ascorbique, la procyanidine, la vitamine E, le glutathion et l'acide lipoïque.

3. Dispositif implantable absorbable à base de fer selon la revendication 1, le promoteur d'absorption de fer présentant un poids moléculaire moyen en poids compris entre 1 000 et 500 000.

4. Dispositif implantable absorbable à base de fer selon la revendication 1, le promoteur d'absorption de fer étant un polymère de procyanidine.

5. Dispositif implantable absorbable à base de fer selon la revendication 1, le promoteur d'absorption de fer comprenant en outre au moins une unité structurelle polymère dégradable.

6. Dispositif implantable absorbable à base de fer selon la revendication 1, le promoteur d'absorption de fer comprenant en outre au moins une unité structurelle polymère non dégradable.

7. Dispositif implantable absorbable à base de fer selon la revendication 5, le polymère dégradable étant au moins l'un des polymères choisis dans le groupe constitué par : le polyester dégradable, le polyaminoacide, le polyanhydride et le glucane.

8. Dispositif implantable absorbable à base de fer selon la revendication 7, le polyester dégradable étant au moins l'un des polyesters choisis dans le groupe constitué par : l'acide polylactique, l'acide polyglycolique, le polysuccinate, le poly(β-hydroxybutyrate), la polycaprolactone, et le polyéthylène adipate.

9. Dispositif implantable absorbable à base de fer selon la revendication 7, le polyaminoacide étant au moins l'un des polyaminoacides choisis dans le groupe constitué par : la polylysine, l'acide polyaspartique, la polycystéine, la polyméthionine, la polyornithine, et l'acide polyglutamique.

10. Dispositif implantable absorbable à base de fer selon la revendication 7, le polyanhydride étant au moins l'un des polyanhydrides choisis dans le groupe constitué par : l'anhydride polyoxalate, l'anhydride polybutanedioïque, l'anhydride polyadipique, l'anhydride polysebacique, l'anhydride polydodécanoïque, l'anhydride polycitrique, l'anhydride polymalique, l'anhydride polysuccinique, l'anhydride polytartrique, l'anhydride polyitaconique et l'anhydride polymaléique.

11. Dispositif implantable absorbable selon la revendication 6, le polymère non dégradable étant au moins l'un des polymères choisis dans le groupe constitué par : le polyéthylène glycol, le polyacrylate, le polysuccinate, le polycarbonate, le polyéthylène-acétate de vinyle, le poly(méthacrylate de n-butyle), le polystyrène, le polyfluorure de vinylidène, la polypyrrolidone de vinyl et le polyhexafluoroéthylène.

12. Dispositif implantable absorbable à base de fer selon la revendication 1, le rapport de masse entre le promoteur d'absorption de fer et le fer dans le substrat à base de fer étant compris entre 0,15:1 et 1:1.

13. Dispositif implantable absorbable à base de fer selon la revendication 1, le promoteur d'absorption de fer étant fixé à une surface du substrat à base de fer ou le promoteur d'absorption de fer étant rempli à l'intérieur du substrat à base de fer.

14. Dispositif implantable absorbable à base de fer selon la revendication 1, le dispositif implantable absorbable à base de fer étant une endoprothèse cardiovasculaire, une endoprothèse cérébrovasculaire, une endoprothèse vasculaire périphérique, une endoprothèse biliaire, une endoprothèse oesophagienne, une endoprothèse des voies aériennes, un dispositif d'occlusion ou un implant orthopédique.

15. Dispositif implantable absorbable à base de fer selon la revendication 1, le dispositif implantable absorbable à base de fer comprenant en outre un médicament actif fixé au substrat à base de fer, le médicament actif étant au moins un médicament choisi parmi les médicaments antiangiogéniques, les médicaments antiplaquettaires, les médicaments antithrombotiques, les médicaments anti-inflammatoires et les médicaments anti-sensibilisants.
